# EUROPEAN PATENT APPLICATION

(11) **EP 4 124 861 A1**
(43) Date of publication of application: **01.02.2023**
(21) Application number: 21000210.1
(22) Date of filing: 31.07.2021
(51) Int. Cl.: G01N 33/50, G01N 33/68

(54) **PERIPHERAL BLOOD MONONUCLEAR CELLS (PBMC) PHENOTYPES AS BIOMARKERS FOR PATIENTS WITH ALZHEIMER'S DISEASE AND/OR MILD COGNITIVE IMPAIRMENT (MCI)**

(71) Applicant: Deutsches Zentrum für Neurodegenerative Erkrankungen e.V. (DZNE), 53127 Bonn (DE)
(72) Inventor: FAVA, Eugenio, 53129 Bonn (DE); SCHNEIDER, Anja, 53123 Bonn (DE); ROTH, Wera, 53343 Wachtberg (DE); DHOLE, Pranjal, 53115 Bonn (DE); BLERSCH, Josephine, 54579 Üxheim-Flesten (DE); DENNER, Philip, 53177 Bonn (DE)
(74) Representative: Krauss, Jan

(57) **Abstract**

The present invention relates to a method for identifying whether a mammal suffers from or is likely to suffer from Alzheimer's disease (AD) and/or a mild cognitive impairment (MCI), using a phenotypic signature as generated using multivariate classification algorithms. Moreover, the present invention relates to a method for stratifying a mammal into a healthy group or a group suffering or likely to suffer from Alzheimer's disease (AD) and/or a mild cognitive impairment (MCI) for a treatment against said disease. In addition, the present invention relates to a method for monitoring the progression of Alzheimer's disease (AD) and/or mild cognitive impairment (MCI) and/or the treatment state thereof in a mammal being treated against said Alzheimer's disease (AD) and/or mild cognitive impairment (MCI). The present invention also relates to a marker panel for identifying whether a mammal suffers from or is likely to suffer from Alzheimer's disease (AD) and/or a mild cognitive impairment (MCI), comprising PBMC-markers as identified. Furthermore, the present invention relates to a use of said marker panel and/or phenotypic signature for diagnostic and/or drug de-risking applications.

## Description

### FIELD OF THE INVENTION

The present invention relates to a method for identifying whether a mammal suffers from or is likely to suffer from Alzheimer's disease (AD) and/or a mild cognitive impairment (MCI), using a phenotypic signature as generated using multivariate classification algorithms. Moreover, the present invention relates to a method for stratifying a mammal into a healthy group or a group suffering or likely to suffer from Alzheimer's disease (AD) and/or a mild cognitive impairment (MCI) for a treatment against said disease. In addition, the present invention relates to a method for monitoring the progression of Alzheimer's disease (AD) and/or mild cognitive impairment (MCI) and/or the treatment state thereof in a mammal being treated against said Alzheimer's disease (AD) and/or mild cognitive impairment (MCI). The present invention also relates to a marker panel for identifying whether a mammal suffers from or is likely to suffer from Alzheimer's disease (AD) and/or a mild cognitive impairment (MCI), comprising PBMC-markers as identified. Furthermore, the present invention relates to a use of said marker panel and/or phenotypic signature for diagnostic and/or drug de-risking applications.

### BACKGROUND

Alzheimer's disease and other dementia are serious morbidities affecting a large number of people worldwide. Those diseases represent social challenge and at the moment no treatment is available. Diagnosis of neurodegenerative disease as well as clinical trial studies have been hindered, in part, by the limited number of biomarker tests that can correctly diagnose these diseases, adequately stratify and enrich cohorts for patients with target pathology, reliably monitor disease progression, and the lack of affordable and non-invasive tests.

There is a growing number of scientific studies where it has been shown that inflammatory processes in the brain are involved in the development of chronic neurodegenerative diseases and that systemic/peripheral immune responses contribute to disease progression. Indeed, the brain does not keep an absolute immune privilege and peripheral inflammatory mediators, such as cytokines, danger signals or other signaling molecules in addition to infiltrating peripheral immune cells can reach the brain and the central nervous system (CNS) (Cunningham et al. 2013, Heppner et al. 2015, McManus and Heneka 2017). Peripheral inflammatory events and infections, such as respiratory tract infections, bacterial, viral and fungal infections are found to be associated with and significantly contribute to the cognitive decline and progression of dementia (McManus and Heneka 2017, Giridharan et al. 2019, Paouri and Georgopoulos 2019). Employing a biocomputational systems level approach based on constructed molecular networks and by the integration of preclinical, genetic and bioinformatic data a strong contribution of immune-regulatory pathways was assigned to late-onset Alzheimer's disease (Zhang et al. 2013).

For inflammation-related diseases and in particular Alzheimer's disease CSF biomarkers are successfully used in clinical diagnosis with high accuracy. However, there is the demanding need for a minimally invasive, cost-effective and reliable method to diagnose early-stage Alzheimer's disease (Blennow 2017, Zetterberg and Burnham 2019).

Recently, ultrasensitive immunoassays have been developed based on immunoprecipitation-mass spectrometry and have successfully been used to measure plasma Abeta biomarkers and to discriminate between healthy controls and patients with AD or mild cognitive impairment (MCI) (Nakamura et al. 2018). The authors could demonstrate that the assessment of plasma APP/Abeta and Abeta40/42 ratios allow to accurately predicting brain Abeta burden (Nakamura et al. 2018, Baldacci et al. 2019, Zetterberg 2019).

Beyond that, it was recently shown that high-precision plasma Abeta42/40 ratio could accurately diagnose brain amyloidosis and therefore can be used to screen cognitively normal individuals to identify at-risk individuals for AD dementia (Schindler et al. 2019, Bendlin et al. 2019). Moreover, plasma neurofilament light chain (NfL) emerged in recent times as a reliable peripheral biomarker of neurodegenerative disease (Baldacci et al. 2019, Zetterberg 2019) showing a good diagnostic accuracy in differentiating neurodegenerative patients from controls and starting from early stages of disease progression (Khalil et al. 2018). Indeed Preische et al. could demonstrate that CSF NfL and serum NfL levels correlated and were found to be elevated at pre-symptomatic stages of familial Alzheimer's disease (Preische et al. 2019), Thus, serum NfL assessment could predict disease progression and brain neurodegeneration at the early pre-symptomatic stages of familial AD. Altogether these facts indicate that blood-based biomarker tests can be possible.

However, there is a huge need for methods that can be used to detect reliable changes, a combination and the analysis of multiple parameters is a necessary step which is supported by the recent expert review by Penner et al. considering biomarkers in blood as a "shadow of events that are occurring in the brain" and expressed as a "fingerprint in blood" worthwhile to follow up in order to overcome the constraint in biomarker development (Penner et al. 2019).

In recent years, a number of multivariate blood-based biomarker panels have been tested and proposed for diagnosing, classifying and prognosing AD, following the hypothesis that the use of panels of markers may outperform single candidate markers (Zetterberg and Burnham 2019).

However, overall there has been a lack of harmonization and replication of the findings (Zetterberg and Burnham 2019). Lately, a hypothesis-led plasma biomarker search was conducted and 53 inflammatory proteins were analyzed in healthy controls, MCI and AD individuals and a collection of analytes and markers of inflammation and complement dysregulation showed significant intergroup differences which could be replicated in an independent cohort (Morgan et al. 2019).

More and more, drugs are to be used in a personalized manner in order to be able to exploit the individual modes of action of a drug in a patient more specifically, and thus to avoid inflammatory reactions. Suitable biomarkers are being sought that allow for a reliable statement that can be made as to whether the drug under consideration is likely to be effective in this patient; whether the specific patient is likely to tolerate the drug under consideration; and how the drug is best dosed for this patient.

Overall, there is a lack of methods for obtaining a robust, reliable, fast, inexpensive and convenient diagnosis of Alzheimer's disease (AD) and/or a mild cognitive impairment (MCI), in particular versus healthy subjects, at the same time allowing the stratification of the patients and subsequent monitoring of the therapy can be carried out, so that potentially inflammation-triggering drugs can be ruled out in advance through drug de-risking.

A considerable challenge of personalized medicine is the analysis of the diagnostic data obtained. For example, genetic data - obtained from procedures such as next-generation sequencing - require computationally complex data processing steps before the actual analysis of the data can take place. The subsequent analysis then requires interdisciplinary cooperation between experts from the medical profession, clinical oncologists, biologists and software engineers. The provision of personalized therapies is therefore associated with a high diagnostic effort.

Currently, automated workflows, disclosed in the prior art are optimized for homogenous readouts or for image-based readouts in human cell lines or murine cell models. These approaches are not able to link intracellular processes on single cell level with extracellular signals of the innate immune response, but this is essential to understand and evaluate the mode of action of perturbation effects in predictive drug discovery and de-risking innate immunity.

The prior art discloses a number of approaches for obtaining and evaluating quantitative data from microscopic imaging.

Loo et al. (Loo LH, Wu LF, Altschuler SJ. Image-based multivariate profiling of drug responses from single cells. Nat Methods. 2007 May;4(5):445-53. doi: 10.1038/nmeth1032. Epub 2007 Apr 1. PMID: 17401369) disclose a multivariate method for the classification of untreated and treated human cancer cells based solely on phenotypic single cell measurements.

Bray et al. (Bray MA, Singh S, Han H, et al. Cell Painting, a high-content image-based assay for morphological profiling using multiplexed fluorescent dyes. Nat Protoc. 2016;11(9):1757-1774. doi:10.1038/nprot.2016.105) detect phenotypic profiling methods based on microscopic images of cell components or organelles to identify biologically relevant similarities and differences between samples based on these profiles and evaluate the effects of chemical, genetic or disease disorders on the phenotypic signature.

Collinet et al. (Collinet C, et al. Systems survey of endocytosis by multiparametric image analysis. Nature. 2010 Mar 11;464(7286):243-9) reveal phenotypic profiling based on multi-parametric image analysis using automated confocal microscopy to analyze the human genome in terms of endocytosis of transferrin and epidermal growth factor.

US 2013-0024130 discloses a method and system for displaying measured values of a complete blood count ("CBC") parameter, the method comprising: displaying the measured values of the CBC parameter obtained from a plurality of samples from a first lot of a quality control composition, wherein the displaying comprises displaying a marker corresponding to each measured value from the first lot on a plot comprising a two dimensional coordinate system, wherein the two dimensional coordinate system comprises a first dimension corresponding to a time at which measured values of the CBC parameter were obtained, and a second dimension corresponding to a numerical value of the CBC parameter.

There has been previous application to use PBMC for specific diseases. For example, US 2018-0251853 (Biomarkers in Peripheral Blood Mononuclear Cells for Diagnosing or Detecting Lung Cancers) uses miRNA present in PBMC to make diagnosis of lung cancer.

EP3803406A1 (Hsp70 protein levels in pbmc samples as biomarker for disease) uses reduced Hsp70 levels in PBMC samples that serve as a biomarker for diseases presenting with a reduced level of Hsp70, such as lysosomal storage diseases, neurodegenerative diseases and muscular diseases. However, all of these methods use single feature marker analysis and no multivariate analysis.

There have been also several attempts to use blood to develop a test for diagnosis of Alzheimer's disease or other dementia. In many cases, those attempts are based on a single marker (e.g., Aβ-42) or a combination of a number of proteins (WO2017223291A1 or US20100167937A1 or US20190219599A1). These methods have been showing limited sensitivity and specificity and lack the capability to be implemented in a scalable and economical manner. Previously, it has also been reported a bioassay using lymphocytes in combination with a specific peptide (WO2018226590A1 (Peptides as biomarkers in the diagnosis, confirmation and treatment of a neurological disorder and immunoprofiling in neurodegenerative disease).

However, methods based only on single-point readout (e.g. blood-based biomarker), such as microscopic phenotypic evaluation, are much more susceptible to failure. Therefore, the present inventors have developed a phenotypic signature/marker panel that allows remedying all the above-mentioned shortcomings in the diagnosis of Ad and MCI, stratification of patients, monitoring the progression of diseases, and de-risking of compounds in a reliable, convenient, reproducible and cost-effective way. The prior art does not disclose or propose comparable assays and panels.

In a first aspect thereof, the invention solves the above problem by providing a phenotypic signature or marker panel for identifying whether a mammal suffers from or is likely to suffer from Alzheimer's disease (AD) and/or a mild cognitive impairment (MCI) or is healthy, comprising PBMC-markers selected from the group consisting of TNFaRel_undefC; IL1bRel_TNFaPosCell; IL1bRel_Bcell; activCell_EstAbsBcell; TNFaRel_Monoc; activCell_EstAbsMonoc; TNFaRel_Tcell; ILl1bRel_actCellsObj; Activationrate_percent; IL1bRel_Monoc; IL1b_pgml; TNFaRel_Bcell; TNFaRel_IL1bPosCell; TNFaRel_TNFaPosCell; IL1bRel IL1bPosCell; TNFa_pos_percent; IL1bRel_Tcell; activCell_EstAbsTcell; IL1bPosCells_Monocyte; IL1b_pos_percent; IL1bRel_undefC; TNFaCells_IL1bCells; activCell_EstAbsUndef; TNFaPosCells_Monocyte; and TNFa_pgml as disclosed herein.

Preferably, the invention solves the above problem by providing a phenotypic signature or marker panel for identifying whether a mammal suffers from or is likely to suffer from Alzheimer's disease (AD) and/or a mild cognitive impairment (MCI) or is healthy, comprising PBMC-markers consisting at least of IL1bRel_Tcell; activCell_EstAbsTcell; IL1bPosCells_Monocyte; IL1b_pos_percent; IL1bRel_undefC; TNFaCells IL1bCells; activCell_EstAbsUndef; TNFaPosCells_Monocyte; TNFa_pgml; optionally including TNFaRel_undefC; IL1bRel_TNFaPosCell; IL1bRel_Bcell, and activCell_EstAbsBcell as disclosed herein.

The markers of the phenotypic signature were determined based on an analysis of a selected set of microscopy-derived features, and biochemical and protein markers of the PBMCs, as described below. Phenotypic signatures were obtained as described herein identifying patients and/or patients groups suffering from of Alzheimer's disease (AD) and/or mild cognitive impairment (MCI) versus healthy, i.e. AD, AD/MCI (MCI with AD indication, also designated herein as MCI-AD or MCI-to-AD, see, for example, Varatharajah, Y., Ramanan, V.K., Iyer, R. et al. Predicting Short-term MCI-to-AD Progression Using Imaging, CSF, Genetic Factors, Cognitive Resilience, and Demographics. Sci Rep 9, 2235 (2019). https://doi.org/10.1038/s41598-019-38793-3), MCI (i.e. MCI-noAD), and healthy.

In a second aspect thereof, the invention solves the above problem by providing a kit, comprising the marker panel and/or phenotypic signature according to the present invention, together with auxiliary agents for performing the method according to the present invention as described herein, preferably with suitable buffers, dyes and/or labels.

In a third aspect thereof, the invention solves the above problem by providing an *in vitro* method for identifying whether a mammal suffers from or is likely to suffer from Alzheimer's disease (AD) and/or a mild cognitive impairment (MCI) or is healthy, said method comprising: i) Providing at least one biological sample obtained from said mammal comprising peripheral blood mononuclear cells (PBMCs) in a suitable liquid medium, iiStimulating said PBMCs in said at least one biological sample with an agent selected from a) no stimulation (i.e. naive or untreated, b) Toll-like receptor activation (e.g. lipopolysaccharide, LPS), c) NOD-Like receptor activation (i.e. ATP as NLRP3 activator), and d) combined Toll-like receptor activation (e.g. LPS and NOD-Like receptor activation (e.g. ATP), iii) Analyzing phenotypic markers selected from the group comprising the markers TNFaRel_undefC; IL1bRel_TNFaPosCell; IL1bRel_Bcell; activCell_EstAbsBcell; TNFaRel_Monoc; activCell_EstAbsMonoc; TNFaRel_Tcell; ILl1bRel_actCellsObj; Activationrate_percent; IL1bRel_Monoc; IL1b_pgml; TNFaRel_Bcell; TNFaRel_IL1bPosCell; TNFaRel TNFaPosCell; IL1bRel_IL1bPosCell; TNFa_pos_percent; IL1bRel_Tcell; activCell_EstAbsTcell; IL1bPosCells_Monocyte; IL1b_pos_percent; IL1bRel_undefC; TNFaCells_IL1bCells; activCell_EstAbsUndef; TNFaPosCells_Monocyte; and TNFa_pgml as disclosed herein in the PBMCs in order to generate at least one phenotypic signature for said PBMCs for said agent; and iv) identifying, based on the analysis of the phenotypic markers and using multivariate classification algorithms, if said mammal suffers from or is likely to suffer from Alzheimer's disease (AD) or a mild cognitive impairment (MCI), or is healthy.

In a preferred embodiment of the method according to the present invention, said method is for identifying whether a mammal suffers from or is likely to suffer from Alzheimer's disease (AD) and/or a mild cognitive impairment (MCI), wherein the stimulation in step ii) is LPS stimulation, wherein the phenotypic markers as analyzed in step iii) are at least comprising IL1bRel_Tcell; activCell_EstAbsTcell; IL1bPosCells_Monocyte; IL1b_pos_percent; IL1bRel_undefC; TNFaCells_IL1bCells; activCell_EstAbsUndef; TNFaPosCells Monocyte; and TNFa_pgml; optionally including TNFaRel_undefC; IL1bRel_TNFaPosCell; IL1bRel_Bcell, and activCell_EstAbsBcell, and identifying, based on the analysis of the phenotypic markers and using multivariate classification algorithms, if said mammal suffers from or is likely to suffer from Alzheimer's disease (AD) and/or a mild cognitive impairment (MCI).

The markers of the phenotypic signature are determined based on analyzing of the phenotypic markers in the cells comprising a suitable staining of microscopically evaluable intracellular and/or extracellular structures of the PBMCs, and subsequent microscopic evaluation of said stained intracellular and/or extracellular structures, and analyzing of the phenotypic markers in the medium comprising a suitable identification of soluble marker proteins and/or their biological activities.

In a fourth aspect thereof, the invention solves the above problem by providing an An *in vitro* method for stratifying a mammal into a healthy group or a group suffering or likely to suffer from Alzheimer's disease (AD) and/or a mild cognitive impairment (MCI) or is healthy, comprising performing the method according to the present invention, and furthermore v) stratifying the mammal into a healthy group or a group suffering or likely to suffer from Alzheimer's disease (AD) and/or a mild cognitive impairment (MCI) or is healthy based on the analysis of step iv).

In a fifth aspect thereof, the invention solves the above problem by providing an *in vitro* method for monitoring the progression of Alzheimer's disease (AD) and/or mild cognitive impairment (MCI) and/or the treatment state thereof in a mammal being treated against said Alzheimer's disease (AD) and/or mild cognitive impairment (MCI), comprising: i) Providing a first biological sample obtained at a first point in time from said mammal being treated comprising peripheral blood mononuclear cells (PBMCs), ii) Providing a second biological sample obtained at a second point in time from the same mammal being treated comprising peripheral blood mononuclear cells (PBMCs), iii) Performing the method according to the present invention on both the first sample and the second sample, and iv) monitoring the progression of Alzheimer's disease (AD) or mild cognitive impairment (MCI) and/or the treatment state thereof in said mammal based on the changes as analyzed in the first and second phenotypic signatures.

In a sixth aspect thereof, the invention solves the above problem by providing an *in vitro* method for identifying a compound for the prevention and/or treatment of Alzheimer's disease (AD) or mild cognitive impairment (MCI) in a mammal, comprising: i) Providing at least one biological sample obtained from said mammal comprising peripheral blood mononuclear cells (PBMCs) in a suitable liquid medium, ii) Stimulating said PBMCs in said at least one biological sample with an agent selected from a) no stimulation (i.e. naïve or untreated, b) Toll-like receptor activation (e.g. lipopolysaccharide, LPS), c) NOD-Like receptor activation (i.e. ATP as NLRP3 activator), and d) combined Toll-like receptor activation (e.g. LPS and NOD-Like receptor activation (e.g. ATP), iii) Suitably contacting said sample with at least one candidate compound, iv) Analyzing phenotypic markers selected from the group comprising TNFaRel_undefC; IL1bRel_TNFaPosCell; IL1bRel_Bcell; activCell_EstAbsBcell; TNFaRel Monoc; activCell_EstAbsMonoc; TNFaRel_Tcell; IL1bRel_actCellsObj; Activationrate_percent; IL1bRel_Monoc; IL1b_pgml; TNFaRel_Bcell; TNFaRel_IL1bPosCell; TNFaRel_TNFaPosCell; IL1bRel_IL1bPosCell; TNFa_pos_percent; IL1bRel_Tcell; activCell_EstAbsTcell; IL1bPosCells_Monocyte; IL1b_pos_percent; IL1bRel_undefC; TNFaCells_IL1bCells; activCell_EstAbsUndef; TNFaPosCells_Monocyte; and TNFa_pgml as disclosed herein in said PBMCs in order to generate at least one phenotypic signature for said PBMCs for said agent, and v) Identifying said compound based on changes of the markers as identified after comparing, using multivariate classification algorithms, the analysis in step iv) with an analysis of said phenotypic markers in a sample in the absence of said candidate compound and/or with an analysis of said phenotypic markers in a control sample.

In a preferred embodiment of the method according to the present invention, said method is for identifying a compound for the prevention and/or, preferably or, treatment of Alzheimer's disease (AD) and/or a mild cognitive impairment (MCI), wherein the stimulation in step ii) is LPS stimulation, wherein the phenotypic markers as analyzed in step iii) are at least comprising IL1bRel_Tcell; activCell_EstAbsTcell; IL1bPosCells_Monocyte; IL1b_pos_percent; IL1bRel_undefC; TNFaCells_IL1bCells; activCell_EstAbsUndef; TNFaPosCells_Monocyte; and TNFa_pgml; optionally including TNFaRel_undefC; IL1bRel_TNFaPosCell; IL1bRel_Bcell, and activCell_EstAbsBcell, and identifying, based on the analysis of the phenotypic markers and using multivariate classification algorithms, if said mammal suffers from or is likely to suffer from Alzheimer's disease (AD) and/or, preferably or, a mild cognitive impairment (MCI).

In a seventh aspect thereof, the invention solves the above problem by providing the use of a marker panel according to the present invention for identifying whether a mammal suffers from or is likely to suffer from Alzheimer's disease (AD) and/or a mild cognitive impairment (MCI) or is healthy, according to the present invention, for stratifying a mammal into a healthy group or a group suffering or likely to suffer from Alzheimer's disease (AD) and/or a mild cognitive impairment (MCI) according to the present invention, for monitoring the progression of Alzheimer's disease (AD) and/or mild cognitive impairment (MCI) and/or the treatment state thereof in a mammal according to the present invention, or for identifying a compound for the prevention and/or treatment of Alzheimer's disease (AD) and/or mild cognitive impairment (MCI) in a mammal according to the present invention.

In the following, the technical features of the present invention will be described further. These features are listed with specific embodiments; however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

The present invention provides a phenotypic signature and/or marker panel as well as *in vitro* methods for diagnostic applications to identify patients suffering from of Alzheimer's disease (AD) and/or mild cognitive impairment (MCI), to stratify patients, to monitor the progression of Alzheimer's disease (AD) and/or mild cognitive impairment (MCI) during treatment of patients, and to screen for efficient treatments and/or drugs. "Alzheimer's disease (AD) and/or mild cognitive impairment (MCI)" as mentioned herein includes the disease phenotypes AD, AD/MCI (MCI with AD indication, also designated herein as MCI-AD or MCI-to-AD, see, for example, Varatharajah, Y., Ramanan, V.K., Iyer, R. et al. Predicting Short-term MCI-to-AD Progression Using Imaging, CSF, Genetic Factors, Cognitive Resilience, and Demographics. Sci Rep 9, 2235 (2019). https://doi.org/10.1038/s41598-019-38793-3), and MCI itself (i.e. MCI-noAD).

PBMC are a constitutive part of the immune system and consist of lymphocytes (T cells, B cells, NK cells) and monocytes. PBMC can be obtained in a large number from a venous blood collection and are routinely produced in many analytical laboratories. Therefore, the inventors consider PBMC as a valuable and reliable source for cell-based biomarkers. In our invention we are using a PBMC cell-based functional assay in combination with predictive analytics methods using machine learning and artificial intelligence (AI) algorithms to deep profile the PBMC obtained by an individual towards a proprietary database containing a statistical relevant sample of the population.

The phenotypic markers as obtained in the present invention represent the numerical vector that reflects the inventive phenotypic signature of the PBMC, and provides a much more robust representation of a mammal's state regarding Alzheimer's disease (AD) and/or mild cognitive impairment (MCI), in particular versus the healthy state, since measurement errors are distributed on multiple readouts compared to above mentioned conventional assays based on single-point readout.

The phenotypic markers were obtained in the present invention by stimulating PBMCs in a biological sample obtained from healthy or patients suffering from of Alzheimer's disease (AD) and/or mild cognitive impairment (MCI). The PBMCs are contacted with inducing or repressing agent of the innate immune response, separated as stimulated from at least part of the supernatant of said biological sample, and at least part of the supernatant was collected and analyzed for factors that induce or repress the innate immunity as released from said cells. In addition, microscopically evaluable intracellular and/or extracellular structures of said PBMCs were suitably stained, and subsequently analyzed by microscopic evaluation of said stained intracellular and/or extracellular structures.

Preferred is a method according to the present invention, wherein the agent is selected from the group i) no stimulation (naive or untreated, ii) Toll-like receptor activation (e.g. lipopolysaccharide, LPS), iii) NOD-Like receptor activation (e.g. ATP, LeuLeuOMe or Nigericin, all of them NLRP3 inflammasome activators), and iv) combined Toll-like receptor activation (e.g. LPS and NOD-Like receptor activation (e.g. ATP, LeuLeuOMe or Nigericin).

After analyzing both of said factors as released into the supernatant and said microscopic evaluation using multivariate classification algorithms a phenotypic signature was obtained as described herein identifying patients and/or patients groups suffering from of Alzheimer's disease (AD) and/or mild cognitive impairment (MCI) versus healthy, i.e. AD, AD/MCI (MCI with AD indication, also designated herein as MCI-AD), MCI (i.e. MCI-noAD), and healthy. The present invention is based on the cell-based marker set and/or biochemical markers and/or genetic markers, combined with a functional assay probing the immune system as described herein. The resulting 25, preferably 6, features selected from the readout of the assay are constituting the "phenotypic signature" or "marker panel" of the individuals' PBMCs according to the present invention.

As used herein, the term "phenotypic signature" covers a multivariate data set of PBMCs of interest in form of a numerical vector obtained by the methods according to the present invention. A phenotypic signature consists at least of the data from the analysis of released factors in the supernatant (such as, for example, TNF-alpha, and IL-1-beta) and data from the microscopic evaluation (such as, for example, chromatin, PYCARD, IL1beta, TNF-alpha and CD-marker staining).

A machine learning multivariate classification algorithm processes the data set obtained by the methods according to the present invention and out of this generates the numerical vector representing the phenotypic signature indicating the innate immune response of said PBMC from said mammal or of cells from control samples. The baseline signature of such control samples is composed by data collection of control cells from samples obtained from healthy donors. Once all data has been collected the data points of the vector undergo a statistical quality check and the most robust data points are used in the final analysis. All numerical vectors of analyzed cells are then classified using said machine learning multivariate classification algorithms to finally assign the sample to an innate immune response group (i.e. healthy or diseased). Preferably, the identification of the phenotypic markers in the cells comprises generating a cluster map providing distinct p-value-signatures of pairwise comparisons, and evaluating said comparisons for statistical differences, preferably using a p-value < 0.05 or < 0.01, using the Kruskal-Wallis and/or the Games-Howell post-hoc test.

As used herein, the term "multivariate classification algorithms" means the process of identifying similarities between the multivariate data set representing the phenotypic signature of said cells as obtained by the methods according to the present invention and the multivariate data sets representing phenotypic signatures from control cells, phenotypic signatures from untreated cells with known innate immune response, or phenotypic signatures from reference cells with known innate immune response contained in a database. Conventional multivariate classification algorithms are available for the skilled person and may be adapted for the purpose of the present invention. For example, Nicholson et al. reveals machine learning multivariate pattern analysis for classification of posttraumatic stress disorder. Apart from that, there is a variety of further documents disclosed in the prior art (Nicholson, Densmore, Kinnon, Cambridge University Press; Volume 49, Issue 12; September 2019, pp. 2049-2059: Machine learning multivariate pattern analysis predicts classification of posttraumatic stress disorder and its dissociative subtype: a multimodal neuroimaging approach).

As mentioned above, the present invention furthermore solves the above problem by providing an *in vitro* method for identifying whether a mammal suffers from or is likely to suffer from Alzheimer's disease (AD) and/or a mild cognitive impairment (MCI) or is healthy, said method comprising: i) Providing at least one biological sample obtained from said mammal comprising peripheral blood mononuclear cells (PBMCs) in a suitable liquid medium, ii) Stimulating said PBMCs in said at least one biological sample with an agent selected from a) no stimulation, b) Toll-like receptor activation, c) NOD-Like receptor activation, and d) combined Toll-like receptor activation and NOD-Like receptor activation, iii) Analyzing phenotypic markers selected from the group comprising the markers TNFaRel_undefC; IL1bRel_TNFaPosCell; IL1bRel_Bcell; activCell_EstAbsBcell; TNFaRel_Monoc; activCell_EstAbsMonoc; TNFaRel_Tcell; ILl1bRel_actCellsObj; Activationrate_percent; IL1bRel_Monoc; IL1b_pgml; TNFaRel_Bcell; TNFaRel_IL1bPosCell; TNFaRel_TNFaPosCell; IL1bRel_IL1bPosCell; TNFa_pos_percent; IL1bRel_Tcell; activCell_EstAbsTcell; IL1bPosCells_Monocyte; IL1b_pos_percent; IL1bRel_undefC; TNFaCells_IL1bCells; activCell_EstAbsUndef; TNFaPosCells Monocyte; and TNFa_pgml as disclosed herein in the PBMCs in order to generate at least one phenotypic signature for said PBMCs for said agent; and iv) identifying, based on the analysis of the phenotypic markers and using multivariate classification algorithms, if said mammal suffers from or is likely to suffer from Alzheimer's disease (AD) or a mild cognitive impairment (MCI), or is healthy.

The present invention furthermore preferably solves the above problem by providing a method for identifying whether a mammal suffers from or is likely to suffer from Alzheimer's disease (AD) or a mild cognitive impairment (MCI), said method comprising: i) Providing at least one biological sample obtained from said mammal comprising peripheral blood mononuclear cells (PBMCs) in a suitable liquid medium, ii) Stimulating said PBMCs in said at least one biological sample with an agent selected from a) no stimulation, b) LPS, iii) Analyzing phenotypic markers selected from the group comprising the markers IL1bRel_Tcell; activCell_EstAbsTcell; IL1bPosCells_Monocyte; IL1b_pos_percent; IL1bRel_undefC; TNFaCells_IL1bCells; activCell_EstAbsUndef; TNFaPosCells_Monocyte; and TNFa_pgml; optionally including TNFaRel_undefC; IL1bRel_TNFaPosCell; IL1bRel_Bcell, and activCell_EstAbsBcellas disclosed herein in the PBMCs in order to generate at least one phenotypic signature for said PBMCs for said agent; and iv) identifying, based on the analysis of the phenotypic markers and using multivariate classification algorithms, if said mammal suffers from or is likely to suffer from Alzheimer's disease (AD) or a mild cognitive impairment (MCI), or is healthy.

The markers of the phenotypic signature were determined based on an analysis of a selected set of microscopy-derived features, and biochemical and protein markers of the PBMCs, as described below. The identification of soluble marker proteins for the analysis may comprise obtaining cytokine release data, such as TNFa, and IL1b, and the microscopic evaluation may comprise chromatin, PYCARD, IL1beta-, TNF-alpha, and CD-marker staining.

Thus, the present invention provides methods to detect if an individual PBMC phenotype deviates from a healthy population or if an individual PBMC phenotype is associated with a given disease phenotype or if an individual PBMC phenotype for a given disease is responding to a specific treatment. The invention is based on a cell-based marker set and/or biochemical markers and/or genetic markers. The resulting features selected from the readout of the assay constitute the phenotype signature of the individual PBMC. The relevant individual PBMC phenotype signature as obtained is analyzed, i.e. compared and contrasted with a database and associated to a certain risk/disease/treatment group (AD and/or MCI) vs healthy as disclosed herein.

Identifying preferably comprises assigning said phenotypic signature to a risk/disease/treatment group (AD and/or MCI) as disclosed herein and comprises the use of multivariate classification algorithms, wherein a change of said phenotypic signature of said mammal when compared to said phenotypic signature in a healthy mammal identifies a mammal suffering from AD and/or MCI-related disease or being at risk of suffering from AD and/or MCI-related disease.

As used herein the term "biological sample" means preferably a liquid biological sample and thus any mammalian body fluid containing the PBMCs to be analyzed from said mammal, which enables the methods to be carried out in accordance with the present invention. For example, such liquid biological samples may be selected from blood, organ or cell type blood sample but without being limited to it. A liquid biological sample also includes PBMCs in a suitable (nutrient) medium.

The term "in vitro" as used herein, refers to methods that are carried out externally to a mammal and in a controlled artificial environment. For example, the cells (PBMCs) for an "in vitro" method may be taken from said mammal, or may be obtained from (immortal) mammalian cell lines. These methods may be performed, for example, in a test tube or Petri dish, but without being limited to these.

The term "supernatant" as used herein means the nutrient medium required for the cultivation and storage of said PBMCs of interest. The culture supernatant is in direct contact with said cells and thus contains released factors by said cells which are essential for obtaining a phenotypic signature of said cells.

The phenotypic markers that were identified in PBMCs as relevant for a phenotypic signature in the context of AD and/or MCI versus healthy in the present invention are selected from the group comprising the following analyses:

| Marker | Analysis |
|---|---|
| TNFaRel_undefC | TNFalpha release per unclassified cell (pg/ml) |
| IL1bRel_TNFaPosCell | ILlbeta release per TNFalpha-positive cell (pg/ml) |
| IL1bRel_Bcell | IL1beta release per B cell (pg/ml) |
| activCell_EstAbsBcell | Percentage of inflammasome speck-positive (=activated) cells in the context of the B cell population |
| TNFaRel_Monoc; | TNFalpha release per monocyte (pg/ml) |
| activCell_EstAbsMonoc | Percentage of inflammasome speck-positive (=activated) cells in the context of the monocyte population |
| TNFaRel_Tcell | TNFalpha release per T cell (pg/ml) |
| IL1bRel_actCellsObj | ILlbeta release per inflammasome speck-positive (=activated) cell (pg/ml) |
| Activationrate_percent | Percentage of inflammasome speck-positive (=activated) cells in the total cell population |
| IL1bRel_Monoc | ILlbeta release per monocyte (pg/ml) |
| IL1b_pgml | ILlbeta release into the cell culture supernatant (pg/ml) |
| TNFaRel_Bcell | TNFalpha release per B cell (pg/ml) |
| TNFaRel_IL1bPosCell | TNFalpha release per IL1beta-positive cell (pg/ml) |
| TNFaRel_TNFaPosCell | TNFalpha release per TNFalpha-positive cell (pg/ml) |
| IL1bRe1_IL1bPosCell | TNFalpha release per IL1beta-positive cell (pg/ml) |
| TNFa_pos_percent | Percentage of TNFalpha-positive cells in the total cell population |
| IL1bRel_Tcell | IL1beta release per T cell (pg/ml) |
| activCell_EstAbsTcell | Percentage of inflammasome speck-positive (=activated) cells in the context of the T cell population |
| IL1bPosCells_Monocyte | Percentage of IL1beta-positive cells in the monocyte cell population |
| IL1b_pos_percent | Percentage of IL1beta-positive cells in the total cell population |
| IL1bRel_undefC | IL1beta release per unclassified cell (pg/ml) |
| TNFaCells_IL1bCells | Ratio of TNFalpha-positive cells and IL1beta-positive cells |
| activCell_EstAbsUndef | Percentage of inflammasome speck-positive (=activated) cells in the context of the unclassified cell population |
| TNFaPosCells_Monocyte | Percentage of TNFalpha-positive cells in the monocyte cell population |
| TNFa_pgml | TNFalpha release into the cell culture supernatant (pg/ml) |
| Control markers | |
| Bcells_percent | Percentage of B cells in the total cell population |
| Monocytes_percent | Percentage of monocytes in the total cell population |
| Tcells_percent | Percentage of T cells in the total cell population |
| UnclassifiedCells_percent | Percentage of unclassified cells in the total cell population |

The measurements and identifications were performed using assays as described in the art, and as disclosed in, for example, PCT/EP2021/052306, which is particularly incorporated by reference.

Preferred is a subset that was identified in PBMCs as relevant for a phenotypic signature in the context of AD and/or MCI versus healthy in the present invention, selected from the group comprising the following analyses:

| Marker (healthy) | Analysis |
|---|---|
| TNFaRel_undefC | TNFalpha release per unclassified cell (pg/ml) |
| IL1bRel_TNFaPosCell | ILlbeta release per TNFalpha-positive cell (pg/ml) |
| IL1bRel_Bcell | IL1beta release per B cell (pg/ml) |
| activCell_EstAbsBcell | Percentage of inflammasome speck-positive (=activated) cells in the context of the B cell population |
| Marker (diseases) | Analysis |
| IL1bRel_Tcell | IL1beta release per T cell (pg/ml) |
| activCell_EstAbsTcell | Percentage of inflammasome speck-positive (=activated) cells in the context of the T cell population |
| IL1bPosCells Monocyte | Percentage of IL1beta-positive cells in the monocyte cell population |
| IL1b_pos_percent | Percentage of IL1beta-positive cells in the total cell population |
| IL1bRel_undefC | ILlbeta release per unclassified cell (pg/ml) |
| TNFaCells_IL1bCells | Ratio of TNFalpha-positive cells and IL1beta-positive cells |
| activCell_EstAbsUndef | Percentage of inflammasome speck-positive (=activated) cells in the context of the unclassified cell population |
| TNFaPosCells_Monocyte | Percentage of TNFalpha-positive cells in the monocyte cell population |
| TNFa_pgml | TNFalpha release into the cell culture supernatant (pg/ml) |
| Control markers | |
| Bcells _percent | Percentage of B cells in the total cell population |
| Monocytes_percent | Percentage of monocytes in the total cell population |
| Tcells_percent | Percentage of T cells in the total cell population |
| UnclassifiedCells_percent | Percentage of unclassified cells in the total cell population |

As used herein the term "toll-like receptors (TLRs)" means structures of the innate immunity and belong to a group of Pattern-Recognition Receptors (PRRs). TLRs are transmembrane proteins and are used for the recognition of PAMPs (Pathogen-Associated Molecular Patterns). PAMPs are highly conserved, pathogen-specific patterns, which are not found in mammals. Such PAMPs can be, for example, proteins, lipids and carbohydrates, such as, for example, lipopolysaccharides or flagellin that occur on the surface of the microorganisms. In contrast, so called "damage-associated molecular patterns (DAMPs)" are host molecules, such as for example heat shock protein (HSPs), or Interleukin-1 (IL-1). These are endogenous, normally intracellular molecules that enter the extracellular space when a cell is damaged. By binding to the PRRs, an inflammatory reaction is triggered.

The term "NOD-like receptors (NLRs)" as used herein relates to structures of the innate immunity and also belong to a further group of PRRs. In contrast to TLRs, NLRs are present as soluble proteins in the cytosol and are responsible for recognition of intracellular pathogen-associated molecular patterns (PAMPs) that were introduced into the cell via bacterial protein secretion or PAMPs that were taken up by immune cells via phagocytosis. Furthermore, danger associated molecular patterns (DAMPs) were used as well (Yang X, Lin G, Han Z, Chai J. Structural Biology of NOD-Like Receptors. Adv Exp Med Biol. 2019;1172:119-141. doi: 10.1007/978-981-13-9367-9_6. PMID: 31628654.).

In another preferred aspect thereof, the present invention solves the above problem by providing an *in vitro* method for stratifying a mammal into a healthy group or a group suffering or likely to suffer from Alzheimer's disease (AD) and/or a mild cognitive impairment (MCI) or is healthy, comprising performing the method according to the present invention, and furthermore v) stratifying the mammal into a healthy group or a group suffering or likely to suffer from Alzheimer's disease (AD) and/or a mild cognitive impairment (MCI) or is healthy based on the analysis of step iv). Thus, the mammal is stratified into at least one group for a treatment against said Alzheimer's disease (AD) and/or a mild cognitive impairment (MCI), if the phenotypic signature of said mammal deviates from the same phenotypic signature in a healthy mammal.

In another preferred aspect thereof, the present invention solves the above problem by providing an *in vitro* method for monitoring the progression of Alzheimer's disease (AD) and/or mild cognitive impairment (MCI) and/or the treatment state thereof in a mammal being treated against said Alzheimer's disease (AD) and/or mild cognitive impairment (MCI), comprising: i) Providing a first biological sample obtained at a first point in time from said mammal being treated comprising peripheral blood mononuclear cells (PBMCs), ii) Providing a second biological sample obtained at a second point in time from the same mammal being treated comprising peripheral blood mononuclear cells (PBMCs), iii) Performing the method according to the present invention on both the first sample and the second sample, and iv) monitoring the progression of Alzheimer's disease (AD) and/or mild cognitive impairment (MCI) and/or the treatment state thereof in said mammal based on the changes as analyzed in the first and second phenotypic signatures.

"Monitoring" as used in the context of the present invention means the regular provision of said biological sample from said mammal, as described herein, and the performance of the methods according to the present invention to provide a reliable and complete indication of the progression of Alzheimer's disease (AD) and/or mild cognitive impairment (MCI) during treatment based on the phenotypic signature analysis. Suitable intervals can be determined by a skilled person.

During said treatment of said mammal, the influence of said treatment on the progression of the Alzheimer's disease (AD) and/or mild cognitive impairment (MCI), i.e. AD, MCI or AD/MCI, is monitored by a change in said phenotypic signature of said mammal.

Preferred is an embodiment of the method according to the present invention, wherein the drug as used for a treatment induces or represses the activity of a factor selected from the group consisting of Toll-like receptors (TLRs) and/or Nod-like receptors (NLRs).

In another preferred aspect thereof, the present invention solves the above problem by providing an *in vitro* method for identifying a compound for the prevention and/or treatment of Alzheimer's disease (AD) and/or mild cognitive impairment (MCI) in a mammal, comprising: i) Providing at least one biological sample obtained from said mammal comprising peripheral blood mononuclear cells (PBMCs) in a suitable liquid medium, ii) Stimulating said PBMCs in said at least one biological sample with an agent selected from i) no stimulation, ii) Toll-like receptor activation, iii) NOD-Like receptor activation, and iv) combined Toll-like receptor activation and NOD-Like receptor activation, iii) Suitably contacting said sample with at least one candidate compound, iv) Analyzing phenotypic markers selected from the group comprising TNFaRel_undefC; IL1bRel_TNFaPosCell; IL1bRel_Bcell; activCell_EstAbsBcell; TNFaRel_Monoc; activCell_EstAbsMonoc; TNFaRel_Tcell; IL1bRel_actCellsObj; Activationrate_percent; IL1bRel_Monoc; IL1b_pgml; TNFaRel_Bcell; TNFaRel_IL1bPosCell; TNFaRel_TNFaPosCell; ILlbRel_ILlbPosCell; TNFa_pos_percent; ILlbRel_Tcell; activCell_EstAbsTcell; IL1bPosCells_Monocyte; IL1b_pos_percent; IL1bRel_undefC; TNFaCells_IL1bCells; activCell_EstAbsUndef; TNFaPosCells_Monocyte; and TNFa_pgml in the medium of said sample, and/or in the PBMCs in order to generate at least one phenotypic signature for said PBMCs for said agent, and v) Identifying said compound based on changes of the markers as identified after comparing, using multivariate classification algorithms, the analysis in step iv) with an analysis of said phenotypic markers in a sample in the absence of said candidate compound and/or with an analysis of said phenotypic markers in a control sample.

Preferred is the method according to the present invention. wherein the phenotypic markers as analyzed in step iv) are at least comprising IL1bRel_Tcell; activCell_EstAbsTcell; IL1bPosCells_Monocyte; IL1b_pos_percent; IL1bRel_undefC; TNFaCells_IL1bCells; activCell_EstAbsUndef; TNFaPosCells_Monocyte; and TNFa_pgml; optionally including TNFaRel_undefC; IL1bRel_TNFaPosCell; IL1bRel_Bcell, and activCell_EstAbsBcell.

Preferred is an analysis of the phenotypic markers comprising a suitable staining of microscopically evaluable intracellular and/or extracellular structures of the PBMCs, and subsequent microscopic evaluation of said stained intracellular and/or extracellular structures, and an analyzing of the phenotypic markers in the medium comprising a suitable identification of soluble marker proteins, preferably comprising obtaining cytokine release data, such as TNFa, and IL1b, and an microscopic evaluation comprising chromatin, PYCARD, IL1beta-, TNF-alpha, and CD-marker staining.

Further preferred is an identification of the phenotypic markers in the cells comprising generating a cluster map providing distinct p-value-signatures of pairwise comparisons, and evaluating said comparisons for statistical differences, preferably using a p-value < 0.05 or < 0.01, using the Kruskal-Wallis and/or the Games-Howell post-hoc test.

Further preferred is an embodiment of the method according to the present invention, wherein said compound is selected from a drug, a small molecule, a polypeptide, an antibody or fragment thereof, a peptide, a polynucleotide, an oligonucleotide, nanoparticles, a carbohydrate, or lipids.

The term "drug" as used in the context of the present invention includes any substance or pharmaceutical composition intended to cure or prevent disease in a mammal. The drug may be an already known substance or pharmaceutical composition, but it also includes those substances or pharmaceutical compositions which are not yet known. The term "pharmaceutical composition" covers all medical preparations, wherein the substances do not occur in their pure form but in their form as an administrable pharmaceutical composition, since the pure substances are formulated in combination with excipients to form a medicinal product. For example, it may be necessary to coat the compound with, or co-administer the compound with, a material to prevent its inactivation, or the compound may be administered to a mammal in an appropriate carrier, for example, in liposomes, or a diluent. Pharmaceutically acceptable diluents include saline and aqueous buffer solutions, for example to bring the substance in form of tablets, capsules, injections, infusions, solutions, salves or syrups. In addition to the pure substances, excipients or the entire drug may also induce or repress the innate immune response, whereby this inducing or repressing impact can be identified with the methods according to the invention based on the phenotypic signature of said cell from said mammal.

The term "small molecules" as used herein comprises low molecular weight compounds with a low molecular mass. This refers to a class of active substances with a molecular mass not exceeding approximately 800 g-mol-1.

In the context of the present invention the term "protein" is used to denote a polymer composed of amino acid monomers joined by peptide bonds. It refers to a molecular chain of amino acids, and does not refer to a specific length of the product and if required can be modified *in vivo* or *in vitro,* for example by glycosylation, amidation, carboxylation or phosphorylation. Amino acid chains with a length of less than approx. 100 amino acids are called "peptides". The terms "peptides", and "proteins" are included within the definition of "polypeptides". A "peptide bond" is a covalent bond between two amino acids in which the α-amino group of one amino acid is bonded to the α-carboxyl group of the other amino acid. All amino acid or polypeptide sequences, unless otherwise designated, are written from the amino terminus (N-terminus) to the carboxy terminus (C-terminus).

The term "antibody" as referred to herein includes whole, full-length antibodies and any fragment or derivative thereof in which the "antigen-binding portion" or "antigen-binding region" or single chains thereof are retained, such as a binding domain of an antibody specific for the immunogenic active moiety of the conjugate. A naturally occurring "antibody" (immunoglobulin) is a glycoprotein comprising at least two heavy (H) chains and two light (L) chains inter-connected by disulfide bonds. Each heavy chain is comprised of a heavy chain variable region (abbreviated herein as VH) and a heavy chain constant region. Heavy chains are classified as mu, delta, gamma, alpha, or epsilon, and define the antibody's isotype as IgM, IgD, IgG, IgA, and IgE, respectively. The heavy chain constant region is comprised of three domains, CHI, CH2 and CH3. Each light chain is comprised of a light chain variable region (abbreviated herein as VL) and a light chain constant region. The light chain constant region is comprised of one domain, CL. The VH and VL regions can be further subdivided into regions of hyper variability, termed complementarity determining regions (CDRs), interspersed with regions that are more conserved, termed framework regions (FR). Each VH and VL is composed of three CDRs and four FRs arranged from amino-terminus to carboxy-terminus in the following order: FR1, CDR1, FR2, CDR2, FR3, CDR3, FR4. The heavy and light chains form two regions: the Fab (fragment, antigen binding) region, also referred to as the variable (Fv) region, and the Fc (fragment, crystallizable) region. The variable regions (Fv) of the heavy and light chains contain a binding domain that interacts with an antigen. The constant (Fc) regions of the antibodies may mediate the binding to host tissues or factors, including various cells of the immune system (e.g., effector cells) and the first component (Clq) of the classical complement system. The term "Fc" as used herein includes native and mutein forms of polypeptides derived from the Fc region of an antibody. Truncated forms of such polypeptides containing the hinge region that promotes dimerization also are included. Fusion proteins comprising Fc moieties (and oligomers formed therefrom) offer the advantage of facile purification by affinity chromatography over Protein A or Protein G columns. One suitable Fc polypeptide is derived from the human IgG1 antibody.

As used herein, the term "polynucleotide" covers linear biopolymers composed of 20 or more nucleotides with alternating nucleic bases. Nucleic acid polymers with a smaller number of nucleotides are called "oligonucleotides". Such polynucleotides may be, for example, DNA, cDNA, RNA or synthetically produced DNA or RNA or a recombinant produced chimeric nucleic acid molecule comprising one of these nucleic acids alone or in combination.

The term "nanoparticle" as used herein defines particles with a particle size smaller than 0.1 µm (< 100 nm) that are either generated naturally or can be synthetically produced. Such nanoparticles can be, for example, carbon-containing nanoparticles, metal and semi-metal oxides, semiconductors, metal sulphides or polymers, but without being limited to these.

As used herein the term "carbohydrates" covers all carbon compounds, consisting of carbon, hydrogen and oxygen, which may be present as monosaccharides or as polysaccharides.

The term "lipid" as used herein refers to the totality of fats and fat-like substances. Lipids are chemically heterogeneous substances that dissolve poorly in water, but well in non-polar solvents.

In another preferred aspect thereof, the present invention solves the above problem by providing the use of a marker panel according to the present invention for identifying whether a mammal suffers from or is likely to suffer from Alzheimer's disease (AD) and/or a mild cognitive impairment (MCI) according to the present invention, for stratifying a mammal into a healthy group or a group suffering or likely to suffer from Alzheimer's disease (AD) and/or a mild cognitive impairment (MCI) versus healthy according to the present invention, for monitoring the progression of Alzheimer's disease (AD) and/or mild cognitive impairment (MCI) and/or the treatment state thereof in a mammal according to the present invention, or for identifying a compound for the prevention and/or treatment of Alzheimer's disease (AD) and/or mild cognitive impairment (MCI) in a mammal according to the present invention.

Further preferred is an embodiment of the method according to the present invention, wherein in step of analyzing of both said factors as released into the supernatant and said microscopic evaluation in order to obtain a combined phenotypic signature a proteome, transcriptome, lipidome and/or genome of said cells is evaluated, and clinical data are included to supplement said combined phenotypic signature.

As used herein, the term "proteome", refers to the totality of proteins expressed by further single cells obtained from said mammal that are evaluated parallel to the microscopic evaluation of said cell in said liquid biological sample from said mammal according to the methods of the present invention. The expressed proteins may occur as intracellular, extracellular or transmembrane proteins. Many methods for the analysis of these proteins are known to the skilled person. For example, the proteome can be analyzed with Western blot or mass spectrometry, but without being limited to these methods. The amount of cellular proteins may vary depending on the time of measurement.

The term "transcriptome" as used herein, refers to the totality of all genes transcribed in further single cells (i.e. the entirety of all RNA molecules present in said further single cells) obtained from said mammal that are evaluated parallel to the microscopic evaluation of said cell in said liquid biological sample from said mammal according to the methods of the present invention. The transcribed RNA molecules are present intracellularly. There is a number of different methods in the prior art for the analysis of cellular RNA, such as the reverse transcriptase polymerase chain reaction (RT-PCR), but without being limited to this method. The amount of RNA present in a cell can vary depending on the time of measurement.

As used herein, the term "lipidome" refers to the totality of all lipids in further single cells obtained from said mammal that are evaluated parallel to the microscopic evaluation of said cell in said liquid biological sample from said mammal according to the methods of the present invention. There are many methods known to the skilled person, such as mass spectrometry, to analyze the lipidome, but without being limited to this.

The term "genome" as used herein refers to the entirety of the genetic information in further single cells obtained from said mammal that are evaluated parallel to the microscopic evaluation of said cell in said liquid biological sample from said mammal according to the methods of the present invention. The term "genetic information" covers all genetic material contained in the cell nucleus, the mitochondria and the plastids. The prior art provides a number of methods, such as, for example fluorescence in situ hybridization (FISH) or polymerase chain reaction (PCR), but without being limited to these.

The term "peripheral blood mononuclear cells (PBMCs)" as used herein covers mononuclear cells of the blood, which have a round cell nucleus. After providing said liquid biological sample from said mammal, PBMCs from blood, for example, may be purified by centrifugation through a hydrophilic colloid. In the methods according to the present invention, both dead and living cells may be used.

Further provided is an embodiment of the method according to the present invention, wherein said mammal is selected from a mouse, a rat, a monkey, a camel, a llama, a rabbit, a pig, a donkey, a cow, a horse, a sheep, and preferably a human.

In another preferred aspect thereof, the present invention solves the above problem by providing a kit, comprising the marker panel and/or phenotypic signature according to the present invention, together with auxiliary agents for performing the method according to the present invention as described herein, preferably with suitable carriers, such as solid carriers, buffers, dyes and/or labels.

Further preferred is an embodiment of the method according to the present invention, wherein said separating comprises immobilizing said cells on a solid carrier material that is transparent, preferably selected from glass or a plastic material, and preferably chemically inert.

The term "solid carrier" refers to any carrier which is insoluble and chemically inert under the reaction conditions without having an influence on cellular reaction. Furthermore, the carrier must allow the substrate to flow in and out. A number of suitable carriers are known for cell immobilization.

As used herein, the term "immobilizing" refers to the spatial fixation of said cell in the liquid biological sample from said mammal to said solid carrier, for example by the action of chemicals, such as, for example, methanol, ethanol, or acetone, but without being limited to these.

In the method according to the present invention said microscopically evaluable intracellular and/or extracellular structures are stained and the microscopic evaluation is performed optically. The skilled person is familiar with a variety of methods that can be used for the optical detection of cellular structures. For example, neutral dyes are bound by the lipid structures, and alkaline dyes may be used for staining of acidic nucleic acids, but without being limited to these. For evaluation of intracellular and/or extracellular proteins, such as for example, PYCARD, TNF-alpha, CD14, CD19, CD3, IL-1-beta, CASP1, NFkappaB, and NLRP3 conventional immunofluorescence may be applied, using commercially available antibodies that bind specific to the respective proteins. By adding respective second fluorochrome-labelled detection antibodies, the corresponding structures on said proteins may be detected with a fluorescence microscope according to the wavelength range of the respective used fluorochrome.

Yet preferred is an embodiment of the method according to the present invention, wherein said released factors in the supernatant to be analyzed comprise cytokines, such as for example, TNF-alpha, and IL-1-beta, and optionally IL18, IL1alpha, IL1-RA, IL6, IL8, IL10, IL18, CXCL1/GROalpha, CXCL6, MCP1/CCL2, MCP4/CCL13, MIP1alpha/CCL3, MIP1beta/CCL4, ENRAGE, and/or VEGFA. As used herein, the term "released factors" covers all signal molecules that may be secreted or released by said living or death cells into the surrounding supernatant, for example hormones, neurotransmitters, nitric oxide and especially cytokines, but without being limited to these. The term "cytokines", as used in the context of the present invention, covers all proteins secreted by said cells in the course of an immunological or inflammatory reaction. The skilled person knows a number of methods for analysis of released factors, in particular cytokines, such as, for example, enzyme-linked immune absorbent spot (ELISpot), or enzyme-linked immunosorbent assay (ELISA), but without being limited to these.

In yet another preferred embodiment of the method according to the present invention, said microscopic evaluation comprises at least one of morphology, subcellular location of said stained intracellular structures, and marker intensity of said stained intracellular structures of said cells.

The term "morphology" as used in the context of the present invention describes the shape and structure of said cells to be analyzed above the molecular level.

As used herein, the term "subcellular location" means the intracellular position of the stained intracellular structures within said cells or extracellular structures on the surface of the cells.

The term "marker intensity" as used herein, describes the strength in which the detection signal is emitted. The strength of the detection signal may be proportional to the amount of detectable intracellular structures present in said cells.

Preferred is an embodiment of the method according to the present invention, wherein said evaluation of the phenotypic signature is carried out automatically.

The term "automatically" as used herein, refers to autonomous microscopic evaluation by using automated microscopy, as well as autonomous collection, storage, processing and evaluation of further data, as obtained by the methods according to the present invention, by said self-learning multivariate algorithms.

Further preferred is an embodiment of the method according to the present invention, wherein the method comprises a comparison with a control from a database.

The term "comparison" as used herein, means that the numerical vector representing the phenotypic signature as obtained by the methods according to the present invention is searched for overlap with numerical vectors representing the phenotypic signatures of controls stored in said database.

As used herein, the term "controls" covers numerical vectors of phenotypic signatures of untreated control cells obtained from healthy mammals indicating the innate immune response of healthy mammals stored in a database for comparison. The controls enable matching of the numerical vector of the phenotypic signature of said cells of interest with numerical vectors of phenotypic signatures of controls whose immune status or immune response group is known to be healthy. Further preferred is an embodiment of the method according to the present invention wherein said innate immune response is determined based on changes in the phenotypic signature when compared to a control provided by said database.

"Changes" as the term is used herein, means the alteration or deviation of said phenotypic signature from a phenotypic signature from control cells, phenotypic signatures from untreated cells with known innate immune response or phenotypic signatures from reference cells with known innate immune response, as provided by a database, to determine the innate immune response, in particular the inflammasome activation status, of a cell. Each tested biological sample from said mammal is compared to the baseline controls and the multivariate classification algorithm determines the change and assigns the numeric vector, and thus the corresponding sample, to a designated innate immune response group.

In summary, the present invention establishes an *in vitro* diagnostic method that will be an integral part of decision-making for physicians within the diagnosis of AD pathology and/or MCI versus healthy. Here, the present invention supports diagnostic at different levels, such as prevention and screening in healthy subjects, diagnosis, prognosis and stratification in subjects with asymptomatic and symptomatic disease, and in cases of treatment and monitoring thereof in chronic conditions and active disease. Thus, it can be applied in the healthy individual as disease interception method for prevention and screening. It can also be applied in the symptomatic and asymptomatic patients for diagnosis, prognosis and stratification. Finally, it can be applied in the late onset of the disease for choice of best treatment and monitoring of disease progression

The terms "of the [present] invention", "in accordance with the invention", "according to the invention" and the like, as used herein are intended to refer to all aspects and embodiments of the invention described and/or claimed herein.

In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. Preferred is ±5% or ±10%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

All references, patents, and publications cited herein are hereby incorporated by reference in their entirety.

The present invention relates to the following items.
Item 1. An *in vitro* method for identifying whether a mammal suffers from or is likely to suffer from Alzheimer's disease (AD) and/or a mild cognitive impairment (MCI) or is healthy, said method comprising: i) Providing at least one biological sample obtained from said mammal comprising peripheral blood mononuclear cells (PBMCs) in a suitable liquid medium, ii) Stimulating said PBMCs in said at least one biological sample with an agent selected from i) no stimulation, ii) Toll-like receptor activation, iii) NOD-Like receptor activation, and iv) combined Toll-like receptor activation and NOD-Like receptor activation, iii) Analyzing phenotypic markers selected from the group comprising TNFaRel_undefC; IL1bRel_TNFaPosCell; IL1bRel_Bcell; activCell_EstAbsBcell; TNFaRel_Monoc; activCell_EstAbsMonoc; TNFaRel_Tcell; IL1bRel_actCellsObj; Activationrate_percent; IL1bRel_Monoc; IL1b_pgml; TNFaRel_Bcell; TNFaRel_IL1bPosCell; TNFaRel_TNFaPosCell; IL1bRel_IL1bPosCell; TNFa_pos_percent; IL1bRel_Tcell; activCell_EstAbsTcell; IL1bPosCells_Monocyte; IL1b_pos_percent; IL1bRel_undefC; TNFaCells IL1bCells; activCell_EstAbsUndef; TNFaPosCells_Monocyte; and TNFa_pgml in the medium of said sample, and/or in the PBMCs of said sample in order to generate at least one phenotypic signature for said PBMCs for said agent; and iv) identifying, based on the analysis of the phenotypic markers and using multivariate classification algorithms, if said mammal suffers from or is likely to suffer from Alzheimer's disease (AD) or a mild cognitive impairment (MCI), or is healthy.
Item 2. An *in vitro* method for stratifying a mammal into a healthy group or a group suffering or likely to suffer from Alzheimer's disease (AD) and/or a mild cognitive impairment (MCI) or is healthy, comprising performing the method according to Item 1, and furthermore v) stratifying the mammal into a healthy group or a group suffering or likely to suffer from Alzheimer's disease (AD) and/or a mild cognitive impairment (MCI) or is healthy based on the analysis of step iv).
Item 3. An *in vitro* method for monitoring the progression of Alzheimer's disease (AD) and/or mild cognitive impairment (MCI) and/or the treatment state thereof in a mammal being treated against said Alzheimer's disease (AD) and/or mild cognitive impairment (MCI), comprising: i) Providing a first biological sample obtained at a first point in time from said mammal being treated comprising peripheral blood mononuclear cells (PBMCs), ii) Providing a second biological sample obtained at a second point in time from the same mammal being treated comprising peripheral blood mononuclear cells (PBMCs), iii) Performing the method according to Item 1 on both the first sample and the second sample, and iv) monitoring the progression of Alzheimer's disease (AD) and/or mild cognitive impairment (MCI) and/or the treatment state thereof in said mammal based on the changes as analyzed in the first and second phenotypic signatures.
Item 4. The method according to Item 3, wherein said first biological sample was obtained before the beginning of the treatment.
Item 5. The method according to Item 3 or 4, wherein said first biological sample is a control sample, either provided from a healthy patient or group of patients or from a different treated patient or group of patients.
Item 6. The method according to any one of Items 1 to 5, wherein the agent is selected from the group consisting of lipopolysaccharide (LPS), LPS and ATP, Leu Leu, and nigericin.
Item 7. The method according to Item 1 for identifying whether a mammal suffers from or is likely to suffer from Alzheimer's disease (AD) or a mild cognitive impairment (MCI), wherein the stimulation in step ii) is LPS stimulation, wherein the phenotypic markers as analyzed in step iii) are at least comprising IL1bRel_Tcell; activCell_EstAbsTcell; IL1bPosCells_Monocyte; IL1b_pos_percent; IL1bRel_undefC; TNFaCells_IL1bCells; activCell_EstAbsUndef; TNFaPosCells_Monocyte; and TNFa_pgml; optionally including TNFaRel_undefC; IL1bRel_TNFaPosCell; IL1bRel_Bcell, and activCell_EstAbsBcell, and identifying, based on the analysis of the phenotypic markers and using multivariate classification algorithms, if said mammal suffers from or is likely to suffer from Alzheimer's disease (AD) or a mild cognitive impairment (MCI).
Item 8. The method according to Item 7, further comprising a stratification according to claim 2, or a monitoring according to any one of Items 3 to 5.
Item 9. The method according to any one of Items 1 to 8, wherein the analyzing of the phenotypic markers in the cells comprises a suitable staining of microscopically evaluable intracellular and/or extracellular structures of the PBMCs, and subsequent microscopic evaluation of said stained intracellular and/or extracellular structures, and the analyzing of the phenotypic markers in the medium comprises a suitable identification of soluble marker proteins.
Item 10. The method according to claim 9, wherein the identification of soluble marker proteins comprises obtaining cytokine release data, such as TNFa, and IL1b, and the microscopic evaluation comprises chromatin, PYCARD, IL1beta-, TNF-alpha, and CD-marker staining.
Item 11. The method according to any one of claims 1 to 10, wherein the identification of the phenotypic markers in the cells comprises generating a cluster map providing distinct p-value-signatures of pairwise comparisons, and evaluating said comparisons for statistical differences, preferably using a p-value < 0.05 or < 0.01, preferably using the Kruskal-Wallis and/or the Games-Howell post-hoc test.
Item 12. An *in vitro* method for identifying a compound for the prevention and/or treatment of Alzheimer's disease (AD) and/or mild cognitive impairment (MCI) in a mammal, comprising: i) Providing at least one biological sample obtained from said mammal comprising peripheral blood mononuclear cells (PBMCs) in a suitable liquid medium, ii) Stimulating said PBMCs in said at least one biological sample with an agent selected from i) no stimulation, ii) Toll-like receptor activation, iii) NOD-Like receptor activation, and iv) combined Toll-like receptor activation and NOD-Like receptor activation, iii) Suitably contacting said sample with at least one candidate compound, iv) Analyzing phenotypic markers selected from the group comprising TNFaRelundefC; IL1bRel_TNFaPosCell; IL1bRel_Bcell; activCellEstAbsBcell; TNFaRel_Monoc; activCell_EstAbsMonoc; TNFaRel_Tcell; IL1bRel_actCellsObj; Activationrate_percent; IL1bRel_Monoc; IL1b_pgml; TNFaRel_Bcell; TNFaRel_IL1bPosCell; TNFaRel_TNFaPosCell; IL1bRel_IL1bPosCell; TNFa_pos_percent; IL1bRel_Tcell; activCell_EstAbsTcell; IL1bPosCells_Monocyte; IL1b_pos_percent; IL1bRel_undefC; TNFaCells_IL1bCells; activCell_EstAbsUndef; TNFaPosCells_Monocyte; and TNFa_pgml in the medium of said sample, and/or in the PBMCs of said sample, in order to generate at least one phenotypic signature for said PBMCs for said agent, and v) Identifying said compound based on changes of the markers as identified after comparing, using multivariate classification algorithms, the analysis in step iv) with an analysis of said phenotypic markers in a sample in the absence of said candidate compound and/or with an analysis of said phenotypic markers in a control sample.
Item 13. The method according to Item 12, wherein the phenotypic markers as analyzed in step iv) are at least comprising IL1bRel_Tcell; activCell_EstAbsTcell; IL1bPosCells_Monocyte; IL1b_pos_percent; IL1bRel_undefC; TNFaCells_IL1bCells; activCell_EstAbsUndef; TNFaPosCells_Monocyte; and TNFa_pgml; optionally including TNFaRel_undefC; IL1bRel_TNFaPosCell; IL1bRel_Bcell, and activCell_EstAbsBcell.
Item 14. The method according to Item 13, wherein the analyzing of the phenotypic markers is according to any one of Items 9 to 11.
Item 15. The method according to any one of the Items 12 to 14, wherein said compound is selected from a drug, a small molecule, a protein, a peptide, an antibody or fragment thereof, a polynucleotide, an oligonucleotide, nanoparticles, a carbohydrate, and lipids.
Item 16. The method according to any one of the Items 1 to 15, wherein said analysis of said phenotypic signature is carried out automatically, and preferably comprises a comparison with a respective control from a database.
Item 17. A marker panel for identifying whether a mammal suffers from or is likely to suffer from Alzheimer's disease (AD) and/or a mild cognitive impairment (MCI) or is healthy, comprising PBMC-markers selected from the group consisting of TNFaRel_undefC; IL1bRel_TNFaPosCell; IL1bRel_Bcell; activCell_EstAbsBcell; TNFaRel_Monoc; activCell_EstAbsMonoc; TNFaRel_Tcell; IL1bRel_actCellsObj; Activationrate_percent; IL1bRel_Monoc; IL1b_pgml; TNFaRel_Bcell; TNFaRel_IL1bPosCell; TNFaRel_TNFaPosCell; IL1bRel_IL1bPosCell; TNFa_pos_percent; IL1bRel_Tcell; activCell_EstAbsTcell; IL1bPosCells_Monocyte; IL1b_pos_percent; IL1bRel_undefC; TNFaCells_IL1bCells; activCell_EstAbsUndef; TNFaPosCells_Monocyte; and TNFa_pgml, and preferably of IL1bRel_Tcell; activCell_EstAbsTcell; IL1bPosCells_Monocyte; IL1b_pos_percent; IL1bRel_undefC; TNFaCells_IL1bCells; activCell_EstAbsUndef; TNFaPosCells_Monocyte; TNFa_pgml; optionally including TNFaRel_undefC; IL1bRel_TNFaPosCell; IL1bRel_Bcell, and activCell_EstAbsBcell.
Item 18. A kit, comprising the marker panel according to Item 17, together with auxiliary agents for performing the method according to any one of Items 1 to 16, preferably suitable buffers, dyes and/or labels.
Item 19. Use of a marker panel according to Item 17 for identifying whether a mammal suffers from or is likely to suffer from Alzheimer's disease (AD) and/or a mild cognitive impairment (MCI) according to Item 1, for stratifying a mammal into a healthy group or a group suffering or likely to suffer from Alzheimer's disease (AD) and/or a mild cognitive impairment (MCI) or healthy according to Item 2, for monitoring the progression of Alzheimer's disease (AD) and/or mild cognitive impairment (MCI) and/or the treatment state thereof in a mammal according to Item 3, or for identifying a compound for the prevention and/or treatment of Alzheimer's disease (AD) and/or mild cognitive impairment (MCI) in a mammal according to any one of Items 12 to 15.

### BRIEF DESCRIPTION OF THE FIGURES

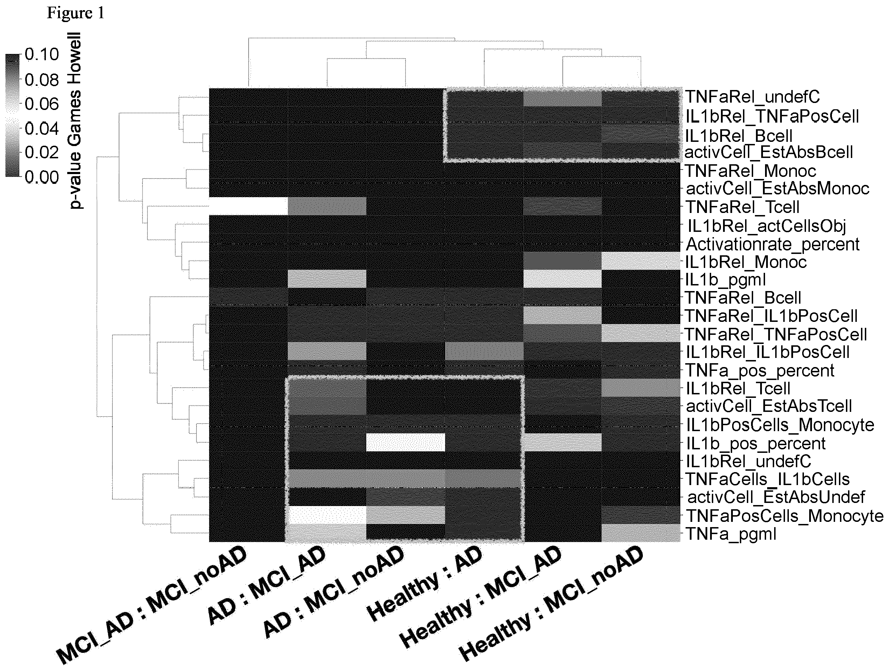

**Figure 1****:** shows the preferred example of the fingerprinting of features (phenotypic signature) as identified according to the present invention for Alzheimer's disease (AD) and/or mild cognitive disease (MCI) toward healthy donors; shown is the fingerprinting of features extracted from the stimulation using LPS (Toll-like receptor activation, also designated as "primed"). For markers, see examples section.

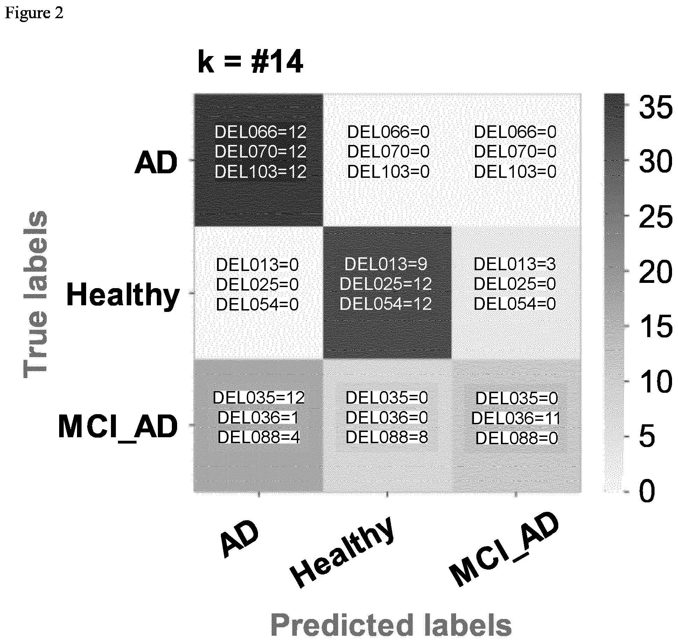

**Figure 2****:** shows an example for a confusion matrix for prediction and classification of AD and/or MCI patients according to the present invention, i.e. AD, MCI, AD/MCI and healthy.

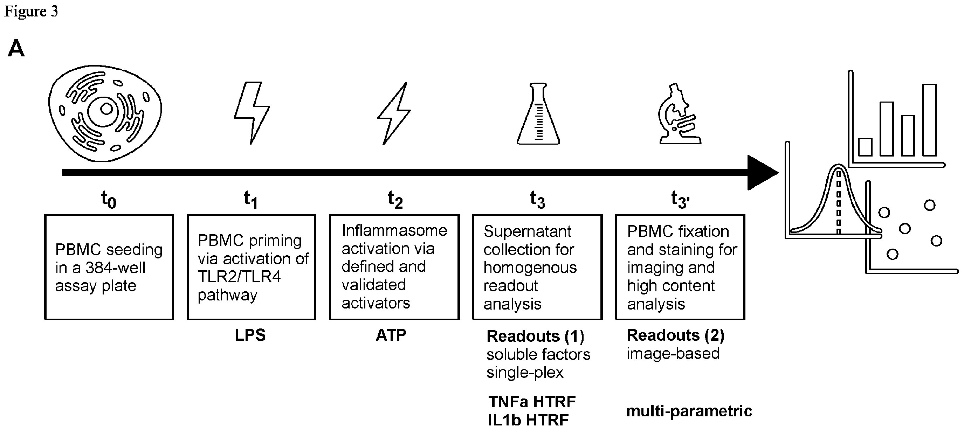

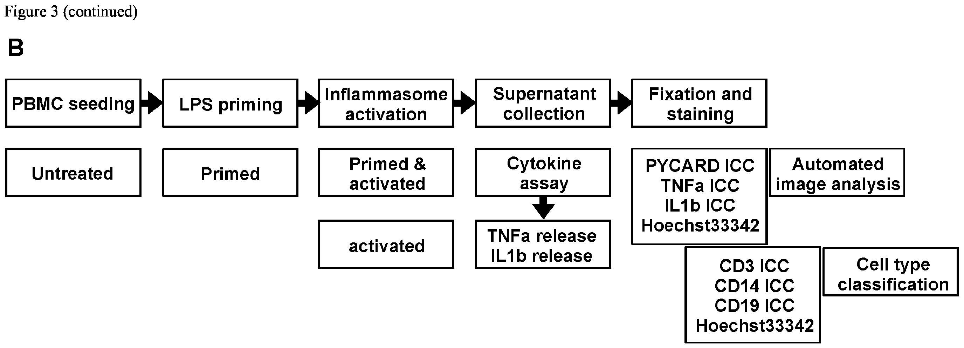

**Figure 3****:** shows in (A) the final cell-based assay format including the time line and the experimental set-up; in (B) the investigated experimental conditions, as specified: i) "untreated" i.e. no stimulation, i.e. naive, ii) "primed", i.e. Toll-like receptor activated, i.e. activators of the TLR2/4 receptor are applied (e.g. LPS), iii) "activated", i.e. NOD-Like receptor activation (e.g. ATP as NLRP3 activator), and iv) "primed and activated", i.e. combined Toll-like receptor activation (e.g. LPS) and NOD-Like receptor activation, i.e. ATP; the assessed homogeneous readouts and the analyzed markers of the immunostaining are represented..

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

### Example

The present invention provides methods to detect if an individual PBMC phenotype deviates from a healthy population or if an individual PBMC phenotype is associated with a given disease phenotype (i.e. AD, MCI, AD/MCI) or if an individual PBMC phenotype for a given disease is responding to a specific treatment against a given disease phenotype (i.e. AD, MCI, AD/MCI). Phenotypic signatures were obtained as described herein identifying patients and/or patients groups suffering from of Alzheimer's disease (AD) and/or mild cognitive impairment (MCI) versus healthy, i.e. AD, AD/MCI (MCI with AD indication, also designated herein as MCI-AD or MCI-to-AD), and healthy.

The present invention is based on the cell-based marker set and/or biochemical markers, combined with a functional assay probing the immune system. The resulting features selected from the readout of the assay are constituting the "phenotypic signature" or "marker panel" of the individuals' PBMCs according to the present invention.

### General method - outline

The inventive method uses human Peripheral Blood Mononuclear Cells (hPBMCs), their phenotypic signatures as obtained by an automated cell-based assay, and finally focuses on the assessment of TLR2/4 signalling and the inflammasome response.

hPBMC were left i) unstimulated (i.e. naive, i.e. untreated) ii) primed (i.e. Toll-like receptor activated) using activators of the TLR2/4 receptor (e.g. LPS), iii) "activated", i.e. NOD-Like receptor activation (e.g. ATP as NLRP3 activator), and iv) "primed and activated", i.e. combined Toll-like receptor activation (e.g. LPS) and NOD-Like receptor activation, i.e. ATP. The priming of the hPBMC induces the activation of specific cellular pathways and trigger the expression of a number of genes involved in the immune response (e.g. TNF-alpha and IL-1beta). Primed hPBMC are capable to mount an inflammasome response upon appropriate stimulation with either Pathogen Associate Molecular Pattern (PAMP) or Danger Associate Molecular Pattern (DAMP). In the present method, DAMP ATP was used to trigger an innate immune response.

The fully automated, robotic-platform-driven cell-based assay was used to seed the cells in a microtiter plate suited for automated image acquisition via confocal microscopy. The PBMCs are investigated under the following experimental conditions: i) no stimulation (naïve or untreated), ii) Toll-like receptor activation (e.g. lipopolysaccharide, LPS), iii) NOD-Like receptor activation (i.e. ATP as NLRP3 activator), and iv) combined Toll-like receptor activation (e.g. LPS) and NOD-like receptor activation (e.g. ATP). Thus, the hPBMC are investigated under the following experimental conditions: 1) naïve (resting state or untreated), 2) primed (mediated by LPS, a Toll-like-receptor (TLR4/TLR2) activator), 3) primed and activated (via LPS as Toll-like-receptor (TLR4/TLR2) activator in combination with the NLRP3 inflammasome inducer ATP) and 4) activated (via the NLRP3 inflammasome inducer ATP). After the given incubation time, at the endpoint of the assay, the cell culture supernatants for each experimental condition were collected, cryopreserved and processed for homogeneous biochemical readout assessment, such as the measurement of cytokine release (e.g. TNF-alpha, IL-1beta) via Homogeneous Time Resolved Fluorescence (HTRF) assay technology.

The hPBMCs on the assay plate are then fixed by paraformaldehyde and processed for immunocytochemistry (ICC) analyses.

The following markers were investigated: (1) chromatin to assess cell nuclei counts and nuclei morphology (associated to the features involving cell count data), (2) IL-1beta to assess the priming and the induction of IL-1beta expression required for the functional inflammasome response (associated to the features involving IL1b-pos-/IL1bPos-related features), (3) TNF-alpha to assess the response to priming (associated to the features involving TNFa-pos-/TNFaPos-related features), and finally, (4) PYCARD (Apoptosis-associated speck-like protein containing a CARD) to assess inflammasome assembly and inflammasome speck formation (associated to the features involving Activationrate-/activCell-related features).

The samples were then imaged using an automated microscope and the resultant images are analysed using an automated image analysis workflow. Figure 3 shows the schematic representation of the assay workflow from cell seeding to the cell fixation passing through the different phases of priming, activation and detection of the assay markers.

The assay readout core was founded on the integration of different readouts including but not limited to cellular phenotype descriptors obtained by high-content-analysis (HCA) of the described immunocytochemistry markers, biochemical readouts including but not limited to cytokine analysis (HTRF assays) and clinical data. At this stage, different experimental quantitative information was collected for the different markers and readouts. The results of the analysis were transformed in a quantitative alpha-numerical data set represented by a collection of individual features (see table below).

The collection of features was conducted for each experimental condition (stimulation), and the combined data were assembled within a single numerical vector representing the phenotypic signature. The phenotypic signature can be combined with other information such as clinical data, anamnesis data etc. Each sample and its phenotypic signature are stored in a database.

Each of the described experimental conditions can elicit either an expected pheno-molecular phenotype or deviations from it. Divergence from the expected phenotype represents a pathological phenotype which is captured by the assay technology. The vector obtained for each patient is expressing the pheno-molecualar signature of the patient or the control. The baseline signature was composed by a collection of healthy donors and this data set represent the baseline to which all the samples tested will be referenced to. After data assembly, all features of the vector underwent statistical analyses to assess the significance of differences of group-wise comparisons for all investigated experimental conditions.

All pheno-molecular vectors were then analysed using a machine learning/artificial intelligence algorithm to finally classify the sample as healthy or diseased. The result of each tested sample was then compared to the baseline controls and the computational methods assigned the sample to either the healthy or the disease class as described herein.

In addition to the patient samples, the database stores information related to sample controls, i.e. individuals that do not have the disease and/or have different diseases. Those samples are constituting the reference set or baseline. Using dedicated machine learning algorithm, each sample was analysed and classified accordingly as a) control, b) Alzheimer's disease (or any other dementia morality under examination) or c) other disease.

### Analysis example

The inventors used microscopy images as input data along with the features obtained from supernatant analysis of the PBMC preparations as analyzed.

The features extracted from microscopic images were obtained as follows: Digital images were analyzed by specific image-analysis software. Objects of interest were segmented within the image. Distinct Regions Of Interest (ROI) were quantified by extracting quantitative parameters, i.e. intensity-based, morphology-based, object-count-based, texture-based, and area-based, giving rise to a primary feature pool. Quantitative secondary features were obtained by combining two or more primary features as necessary.

A subset of extracted features was selected to compose the base vector for the phenotypic signature. In each treatment group, the features were evaluated for statistical differences (p-value < 0.05) using the Kruskal-Wallis and Games-Howell post-hoc tests. These tests are non-parametric statistic tests and were used to provide evidence of differences among the median of three or more independent groups in general. The selected features show significant statistical differences between the groups, indicating that the PBMC of the patients and controls react differently for each treatment.

The phenotypic markers/phenotypic signature as identified and analyzed herein were:

| Marker | Analysis |
|---|---|
| TNFaRel_undefC | TNFalpha release per unclassified cell (pg/ml) |
| IL1bRel_TNFaPosCell | IL1beta release per TNFalpha-positive cell (pg/ml) |
| IL1bRel_Bcell | IL1beta release per B cell (pg/ml) |
| activCell_EstAbsBcell | Percentage of inflammasome speck-positive (=activated) cells in the context of the B cell population |
| TNFaRel_Monoc; | TNFalpha release per monocyte (pg/ml) |
| activCell_EstAbsMonoc | Percentage of inflammasome speck-positive (=activated) cells in the context of the monocyte population |
| TNFaRel_Tcell | TNFalpha release per T cell (pg/ml) |
| IL1bRel_actCellsObj | ILlbeta release per inflammasome speck-positive (=activated) cell (pg/ml) |
| Activationrate_percent | Percentage of inflammasome speck-positive (=activated) cells in the total cell population |
| IL1bRel_Monoc | IL1beta release per monocyte (pg/ml) |
| IL1b_pgml | IL1beta release into the cell culture supernatant (pg/ml) |
| TNFaRel_Bcell | TNFalpha release per B cell (pg/ml) |
| TNFaRel_IL1bPosCell | TNFalpha release per IL1beta-positive cell (pg/ml) |
| TNFaRel TNFaPosCell | TNFalpha release per TNFalpha-positive cell (pg/ml) |
| IL1bRel_IL1bPosCell | TNFalpha release per IL1beta-positive cell (pg/ml) |
| TNFa_pos_percent | Percentage of TNFalpha-positive cells in the total cell population |
| IL1bRel_Tcell | IL1beta release per T cell (pg/ml) |
| activCell_EstAbsTcell | Percentage of inflammasome speck-positive (=activated) cells in the context of the T cell population |
| IL1bPosCells_Monocyte | Percentage of IL1beta-positive cells in the monocyte cell population |
| IL1b_pos_percent | Percentage of IL1beta-positive cells in the total cell population |
| IL1bRel_undefC | IL1beta release per unclassified cell (pg/ml) |
| TNFaCells_IL1bCells | Ratio of TNFalpha-positive cells and IL1beta-positive cells |
| activCell_EstAbsUndef | Percentage of inflammasome speck-positive (=activated) cells in the context of the unclassified cell population |
| TNFaPosCells_Monocyte | Percentage of TNFalpha-positive cells in the monocyte cell population |
| TNFa_pgml | TNFalpha release into the cell culture supernatant (pg/ml) |
| Control markers | |
| Bcells_percent | Percentage of B cells in the total cell population |
| Monocytes_percent | Percentage of monocytes in the total cell population |
| Tcells_percent | Percentage of T cells in the total cell population |
| UnclassifiedCells_percent | Percentage of unclassified cells in the total cell population |

The measurements and identifications were performed using assays as described in the art, and as disclosed in, for example, PCT/EP2021/052306, which is particularly incorporated by reference.

As shown in Figure 1, a subset of features as described herein was observed as statistically different for the AD patients (box A) while another set of features is statistically significant for the healthy sample (box B). This enables to identify monitor the response of the PBMC for each treatment group based on features that discriminate between healthy and AD/MCI and/or between AD versus MCI. Particularly preferred markers therefore are

| Marker (healthy) | Analysis |
|---|---|
| TNFaRel_undefC | TNFalpha release per unclassified cell (pg/ml) |
| IL1bRel_TNFaPosCell | IL1beta release per TNFalpha-positive cell (pg/ml) |
| IL1bRel_Bcell | ILlbeta release per B cell (pg/ml) |
| activCell_EstAbsBcell | Percentage of inflammasome speck-positive (=activated) cells in the context of the B cell population |
| Marker (diseases) | Analysis |
| IL1bRel_Tcell | IL1beta release per T cell (pg/ml) |
| activCell_EstAbsTcell | Percentage of inflammasome speck-positive (=activated) cells in the context of the T cell population |
| IL1bPosCells_Monocyte | Percentage of IL1beta-positive cells in the monocyte cell population |
| IL1b_pos_percent | Percentage of IL1beta-positive cells in the total cell population |
| IL1bRel_undefC | ILlbeta release per unclassified cell (pg/ml) |
| TNFaCells_IL1bCells | Ratio of TNFalpha-positive cells and IL1beta-positive cells |
| activCell_ EstAbsUndef | Percentage of inflammasome speck-positive (=activated) cells in the context of the unclassified cell population |
| TNFaPosCells_Monocyte | Percentage of TNFalpha-positive cells in the monocyte cell population |
| TNFa_pgml | TNFalpha release into the cell culture supernatant (pg/ml) |
| Control markers | |
| Bcells_percent | Percentage of B cells in the total cell population |
| Monocytes_percent | Percentage of monocytes in the total cell population |
| Tcells_percent | Percentage of T cells in the total cell population |
| UnclassifiedCells_percent | Percentage of unclassified cells in the total cell population |

In order to validate the inventors' approach, PBMC with a specific disease phenotype were analyzed, i.e., Alzheimer's disease (AD). 30 AD patients, 20 Mild cognitive impairment (MCI) patients (MCI group with AD indication), and 35 healthy donors were included. The method successfully assigned the samples from unseen patients to the correct class with a high degree of accuracy even with a limited sample size.

The resulting cluster map provided a distinct p-value-signature (e.g. Games_Howell test-based) for pairwise comparisons of groups (e.g., healthy vs. AD, healthy vs. MCI, etc.). The resulting classification of the different groups put in evidence the differences among the different features within the pathological and healthy samples, thus generating the unique fingerprint (phenotypic signature) for each sample and conditions, enabling an improved diagnosis for the relevant disease in question. This information can be used to establish a diagnosis toward disease or healthy.

In the AD/MCI cohort as examined in the context of the present invention, three different patient classes were established: 1) Healthy, 2) MCI_AD, 3) AD, and 4) MCI no AD. The healthy class corresponds to donors with no disposition to cognitive impairment. The MCI_AD class corresponds to donors with mild cognitive impairment with onset of Alzheimer's disease. The AD class corresponds to donors diagnosed with Alzheimer's disease.

For the machine-learning- based classification via support vector machine algorithms, for each patient, between 4 to 12 replicate samples were tested. Thus, in order to eliminate the sample bias, the inventors randomly sampled data for each patient for replicates such that each patient will have the same number of replicates in the dataset. In order to assess the prediction ability on the new patient, it is important that the training and testing algorithms do not see these patient samples at all during the learning phase; but are instead used only as metric for predictability. Thus, the inventors took out 3 patients from each class that composed the validation dataset. The remaining data was then split randomly in 70-30 ratio as training-testing data.

In order to avoid the algorithm "getting lucky" by random chance, the inventors prepared 20 k-fold validation datasets. This means the dataset is split randomly into train-test-validation sets in the manner mentioned above 20 times and evaluated by the classifier to check for robustness. The k-fold validation allowed to evaluate the uncertainty in whether the collected patient samples make up as the representative samples for the given disease class or whether more samples are required to improve the ability to predict accurately the disease classes. The resulting feature set was the set of the features according to the present invention that gave the highest predictability for predicting in which disease category a given donor belongs to with high confidence for the classifier.

Furthermore, the inventors ranked the robustness of features based on how frequently they were chosen to be robust features in different k-fold datasets. The more frequently occurring features get a higher rank for robustness. The result was a classification for Alzheimer disease, Mild Cognitive Impairment (MCI) patients and healthy individuals, by using the aforementioned method, as shown in Figure 2. Within this approach the present invention suggests a probability of a given PBMC sample to be associated to a given disease. Thus, Figure 2 shows a confusion matrix with the prediction of a given sample to belong either to the healthy population or to one of the disease phenotypes under observation, as described herein (AD and/or MCI).

## Claims

1. An *in vitro* method for identifying whether a mammal suffers from or is likely to suffer from Alzheimer's disease (AD) and/or a mild cognitive impairment (MCI) or is healthy, said method comprising:
i) Providing at least one biological sample obtained from said mammal comprising peripheral blood mononuclear cells (PBMCs) in a suitable liquid medium,
ii) Stimulating said PBMCs in said at least one biological sample with an agent selected from i) no stimulation, ii) Toll-like receptor activation, iii) NOD-Like receptor activation, and iv) combined Toll-like receptor activation and NOD-Like receptor activation,
iii) Analyzing phenotypic markers selected from the group comprising
TNFaRel_undefC; IL1bRel_TNFaPosCell; IL1bRel_Bcell; activCell_EstAbsBcell; TNFaRel_Monoc; activCell_EstAbsMonoc; TNFaRel_Tcell; IL1bRel_actCellsObj; Activationrate_percent; ILlbRel_Monoc; IL1b_pgml; TNFaRel_Bcell; TNFaRel_IL1bPosCell; TNFaRel_TNFaPosCell; IL1bRel_IL1bPosCell; TNFa_pos_percent; IL1bRel_Tcell; activCell_EstAbsTcell; IL1bPosCells_Monocyte; IL1b_pos_percent; IL1bRel_undefC; TNFaCells_IL1bCells; activCell_EstAbsUndef; TNFaPosCells_Monocyte; and TNFa_pgml in the medium of said sample, and/or in the PBMCs of said sample in order to generate at least one phenotypic signature for said PBMCs for said agent; and
iv) identifying, based on the analysis of the phenotypic markers and using multivariate classification algorithms, if said mammal suffers from or is likely to suffer from Alzheimer's disease (AD) or a mild cognitive impairment (MCI), or is healthy.

2. An *in vitro* method for stratifying a mammal into a healthy group or a group suffering or likely to suffer from Alzheimer's disease (AD) and/or a mild cognitive impairment (MCI) or is healthy, comprising performing the method according to claim 1, and furthermore
v) stratifying the mammal into a healthy group or a group suffering or likely to suffer from Alzheimer's disease (AD) and/or a mild cognitive impairment (MCI) or is healthy based on the analysis of step iv).

3. An *in vitro* method for monitoring the progression of Alzheimer's disease (AD) and/or mild cognitive impairment (MCI) and/or the treatment state thereof in a mammal being treated against said Alzheimer's disease (AD) and/or mild cognitive impairment (MCI), comprising: i) Providing a first biological sample obtained at a first point in time from said mammal being treated comprising peripheral blood mononuclear cells (PBMCs), ii) Providing a second biological sample obtained at a second point in time from the same mammal being treated comprising peripheral blood mononuclear cells (PBMCs), iii) Performing the method according to claim 1 on both the first sample and the second sample, and iv) monitoring the progression of Alzheimer's disease (AD) and/or mild cognitive impairment (MCI) and/or the treatment state thereof in said mammal based on the changes as analyzed in the first and second phenotypic signatures, wherein preferably said first biological sample was obtained before the beginning of the treatment.

4. The method according to claim 3, wherein said first biological sample is a control sample, either provided from a healthy patient or group of patients or from a different treated patient or group of patients.

5. The method according to any one of claims 1 to 4, wherein the agent is selected from the group consisting of lipopolysaccharide (LPS), LPS and ATP, Leu Leu, and nigericin.

6. The method according to claim 1 for identifying whether a mammal suffers from or is likely to suffer from Alzheimer's disease (AD) or a mild cognitive impairment (MCI), wherein the stimulation in step ii) is LPS stimulation, wherein the phenotypic markers as analyzed in step iii) are at least comprising IL1bRel_Tcell; activCell_EstAbsTcell; IL1bPosCells_Monocyte; IL1b_pos_percent; IL1bRel_undefC; TNFaCells_IL1bCells; activCell_EstAbsUndef; TNFaPosCells_Monocyte; and TNFa_pgml; optionally including TNFaRel_undefC; IL1bRel_TNFaPosCell; IL1bRel_Bcell, and activCell_EstAbsBcell, and identifying, based on the analysis of the phenotypic markers and using multivariate classification algorithms, if said mammal suffers from or is likely to suffer from Alzheimer's disease (AD) or a mild cognitive impairment (MCI), preferably further comprising a stratification according to claim 2, or a monitoring according to any one of claims 3 to 5.

7. The method according to any one of claims 1 to 6, wherein the analyzing of the phenotypic markers in the cells comprises a suitable staining of microscopically evaluable intracellular and/or extracellular structures of the PBMCs, and subsequent microscopic evaluation of said stained intracellular and/or extracellular structures, and the analyzing of the phenotypic markers in the medium comprises a suitable identification of soluble marker proteins, wherein preferably the identification of soluble marker proteins comprises obtaining cytokine release data, such as TNFa, and IL1b, and the microscopic evaluation comprises chromatin, PYCARD, IL1beta-, TNF-alpha, and CD-marker staining.

8. The method according to any one of claims 1 to 7, wherein the identification of the phenotypic markers in the cells comprises generating a cluster map providing distinct p-value-signatures of pairwise comparisons, and evaluating said comparisons for statistical differences, preferably using a p-value < 0.05 or < 0.01, preferably using the Kruskal-Wallis and/or the Games-Howell post-hoc test.

9. An *in vitro* method for identifying a compound for the prevention and/or treatment of Alzheimer's disease (AD) and/or mild cognitive impairment (MCI) in a mammal, comprising:
i) Providing at least one biological sample obtained from said mammal comprising peripheral blood mononuclear cells (PBMCs) in a suitable liquid medium,
ii) Stimulating said PBMCs in said at least one biological sample with an agent selected from i) no stimulation, ii) Toll-like receptor activation, iii) NOD-Like receptor activation, and iv) combined Toll-like receptor activation and NOD-Like receptor activation,
iii) Suitably contacting said sample with at least one candidate compound,
iv) Analyzing phenotypic markers selected from the group comprising TNFaRel_undefC; IL1bRel_TNFaPosCell; IL1bRel_Bcell; activCell_EstAbsBcell; TNFaRel_Monoc; activCell_EstAbsMonoc; TNFaRel_Tcell; IL1bRel_actCellsObj; Activationrate_percent; IL1bRel_Monoc; IL1b_pgml; TNFaRel_Bcell; TNFaRel_IL1bPosCell; TNFaRel_TNFaPosCell; IL1bRel_IL1bPosCell; TNFa_pos_percent; IL1bRel_Tcell; activCell_EstAbsTcell; IL1bPosCells_Monocyte; IL1b_pos_percent; IL1bRel_undefC; TNFaCells_IL1bCells; activCell_EstAbsUndef; TNFaPosCells_Monocyte; and TNFa_pgml in the medium of said sample, and/or in the PBMCs of said sample, in order to generate at least one phenotypic signature for said PBMCs for said agent, and
v) Identifying said compound based on changes of the markers as identified after comparing, using multivariate classification algorithms, the analysis in step iv) with an analysis of said phenotypic markers in a sample in the absence of said candidate compound and/or with an analysis of said phenotypic markers in a control sample.

10. The method according to claim 9, wherein the phenotypic markers as analyzed in step iv) are at least comprising IL1bRel_Tcell; activCell_EstAbsTcell; IL1bPosCells_Monocyte; IL1b_pos_percent; IL1bRel_undefC; TNFaCells_IL1bCells; activCell_EstAbsUndef; TNFaPosCells_Monocyte; and TNFa_pgml; optionally including TNFaRel_undefC; IL1bRel_TNFaPosCell; IL1bRel_Bcell, and activCell_EstAbsBcell, wherein preferably the analyzing of the phenotypic markers is according to any one of claims 7 or 8.

11. The method according to any one of the claims 9 or 10, wherein said compound is selected from a drug, a small molecule, a protein, a peptide, an antibody or fragment thereof, a polynucleotide, an oligonucleotide, nanoparticles, a carbohydrate, and lipids.

12. The method according to any one of the claims 1 to 11, wherein said analysis of said phenotypic signature is carried out automatically, and preferably comprises a comparison with a respective control from a database.

13. A marker panel for identifying whether a mammal suffers from or is likely to suffer from Alzheimer's disease (AD) and/or a mild cognitive impairment (MCI) or is healthy, comprising PBMC-markers selected from the group consisting of TNFaRel_undefC; IL1bRel_TNFaPosCell; IL1bRel_Bcell; activCell_EstAbsBcell; TNFaRel_Monoc; activCell_EstAbsMonoc; TNFaRel_Tcell; ILl1bRel_actCellsObj; Activationrate_percent; IL1bRel_Monoc; IL1b_pgml; TNFaRel_Bcell; TNFaRel_IL1bPosCell; TNFaRel_TNFaPosCell; IL1bRel_IL1bPosCell; TNFa_pos_percent; IL1bRel_Tcell; activCell_EstAbsTcell; IL1bPosCells_Monocyte; IL1b_pos_percent; IL1bRel_undefC; TNFaCells_IL1bCells; activCell_EstAbsUndef; TNFaPosCells_Monocyte; and TNFa_pgml, and preferably of
IL1bRel_Tcell; activCell_EstAbsTcell; IL1bPosCells_Monocyte; IL1b_pos_percent; IL1bRel_undefC; TNFaCells_IL1bCells; activCell_EstAbsUndef; TNFaPosCells_Monocyte; TNFa_pgml; optionally including TNFaRel_undefC; IL1bRel_TNFaPosCell; ILlbRelBcell, and activCell_EstAbsBcell.

14. A kit, comprising the marker panel according to claim 13, together with auxiliary agents for performing the method according to any one of claims 1 to 12, preferably suitable buffers, dyes and/or labels.

15. Use of a marker panel according to claim 17 for identifying whether a mammal suffers from or is likely to suffer from Alzheimer's disease (AD) and/or a mild cognitive impairment (MCI) according to claim 1, for stratifying a mammal into a healthy group or a group suffering or likely to suffer from Alzheimer's disease (AD) and/or a mild cognitive impairment (MCI) or healthy according to claim 2, for monitoring the progression of Alzheimer's disease (AD) and/or mild cognitive impairment (MCI) and/or the treatment state thereof in a mammal according to claim 3, or for identifying a compound for the prevention and/or treatment of Alzheimer's disease (AD) and/or mild cognitive impairment (MCI) in a mammal according to any one of claims 9 to 12.
